# EUROPÄISCHE PATENTSCHRIFT

(11) **EP 3 103 414 B1**
(45) Veröffentlichungstag und Bekanntmachung des Hinweises auf die Patenterteilung: **27.11.2024**
(21) Anmeldenummer: 16173650.9
(22) Anmeldetag: 09.06.2016
(51) Int. Cl.: A61F 2/16

(54) **LINSENIMPLANTAT ZUM IMPLANTIEREN IN EIN ZU BEHANDELNDES AUGE, BASISELEMENT EINES LINSENIMPLANTATS UND OPTISCH WIRKSAMES LINSENELEMENT EINES LINSENIMPLANTATS**
LENS IMPLANT FOR IMPLANTATION INTO AN EYE TO BE TREATED, LENSE IMPLANT BASE ELEMENT AND OPTICALLY EFFECTIVE LENS ELEMENT OF A LENS IMPLANT
IMPLANT DE LENTILLE DESTINE A ETRE IMPLANTE DANS UN IL A SOIGNER, ELEMENT DE BASE D'UN IMPLANT DE LENTILLE ET ELEMENT DE LENTILLE A ACTION OPTIQUE D'UN IMPLANT DE LENTILLE

(30) Priorität: 11.06.2015 DE 102015007534
(43) Veröffentlichungstag der Anmeldung: 14.12.2016
(73) Patentinhaber: Gerl, Matthias, 48683 Ahaus (DE)
(72) Erfinder: Gerl, Matthias, 48683 Ahaus (DE); Müller, Matthias, 48683 Ahaus (DE)
(74) Vertreter: Frenkel, Matthias Alexander

(56) Entgegenhaltungen:
- EP-A1- 2 851 038
- EP-A1- 3 042 634
- WO-A1-2013/158942
- DE-A1- 19 637 692
- US-A- 5 326 347
- US-A1- 2005 015 144
- US-A1- 2006 047 339
- US-A1- 2006 116 765
- US-A1- 2011 264 210
- US-A1- 2013 190 868
- US-A1- 2013 289 467

## Beschreibung

### Technisches Gebiet

Die vorliegende Offenbarung betrifft ein Linsenimplantat zum Implantieren in ein zu behandelndes Auge.

### Hintergrund

Das menschliche optische System umfasst lichteintrittsseitig eine Hornhaut und eine Linse. Im Idealfall werden Aberrationen höherer Ordnung, welche in der Hornhaut auftreten, durch die Linse kompensiert. Bei einer Kataraktoperation oder bei einem Clear Lens Exchange (CLE) wird die Linse entfernt und durch eine Kunstlinse ausgetauscht. Der Grund hierfür ist, dass im Falle der Kataraktoperation ein Katarakt (grauer Star) vorliegt, welcher zu einer Trübung der Linse führt. Ein Clear Lens Exchange wird hingegen dann durchgeführt, wenn eine besonders starke Fehlsichtigkeit einen Austausch der Linse erforderlich macht. Heutzutage werden hochwertige sphärische oder asphärische, torische und multifokale Kunstlinsen hergestellt, die jedoch nicht in der Lage sind, Aberrationen höherer Ordnung der Hornhaut zu kompensieren. Hierbei besteht ein Problem darin, dass weder eine genaue Ausrichtung der Linse erreicht werden kann, noch eine verlässliche Rotationstabilität hergestellt werden kann. Diese Faktoren sind jedoch unerlässlich, um diese individuellen Irregularitäten der Hornhaut zu kompensieren.

Besonders starke nicht-rotationssymmetrische Irregularitäten der Hornhaut treten bei einem Keratokonus (fortschreitende Ausdünnung und kegelförmige Verformung der Hornhaut) auf, der über einen unregelmäßigen Astigmatismus eine Koma erzeugt.

US 5 326 347 A offenbart ein intraokulares Implantat zur Verwendung in einem menschlichen Auge in Folge eines Katarakt, refraktiver oder anderer Augenchirurgie. Das Implantat hat einen Halter und eine Abbildungsoptik, die zu einer kompakten Konfiguration gefaltet werden, separat in das Auge eingeführt und dann durch den Chirurgen im Auge zusammengebaut werden kann, indem die Optik auf dem Halter montiert wird, nachdem der Halter in das Auge implantiert worden ist. Diese Optik beinhaltet eine Linse, die innerhalb des Auges in Bezug auf den Halter durch den Chirurgen während der Operation einstellbar ist. Die offenbarten Implantate werden in den Kapselsack eines Auges eingeführt, aus dem der Kern und die Rinde entfernt worden sind.

US 2006/047339 offenbart intraokulare Vorrichtungen zur Verwendung und Befestigung an der natürlichen Linsenkapsel eines Auges. Die Linsenkapsel kann in einer Konfiguration gehalten werden, um postoperative Veränderungen zu vermeiden, die schädlich für das Sehen sind. Einzel- oder Dual-Optik-Systeme sind vorgesehen, die aufgenommen werden können. Kombinationen von Vorrichtungen, um Doppeloptiksysteme zu erhalten, werden offenbart.

Weitere bekannte Linsenimplantate werden beispielsweise in den Dokumenten US 2006/116765 A1, US 2013/190868 A1, US 2005/015144 A1, WO 2013/158942 A1, EP 2 851 038 A1, DE 196 37 692 A1 und US 2013/289467 A1 beschrieben.

### Zusammenfassung beispielhafter Ausführungsformen

Es ist wünschenswert, ein verbessertes Linsenimplantat bereitzustellen. Das Linsenimplantat kann beispielsweise auf schonende Art und Weise implantierbar sein.

Die obige Aufgabe wird durch ein Linsenimplantat gemäß Anspruch 1 gelöst.

Ein Aspekt der vorliegenden Offenbarung betrifft ein Linsenimplantat zum Implantieren in ein zu behandelndes Auge, umfassend: ein Basiselement; und ein optisch wirksames Linsenelement, wobei zumindest das Basiselement oder das Linsenelement mindestens ein Befestigungselement zum Befestigen des Linsenelements an dem Basiselement aufweist. Anders ausgedrückt können das Basiselement und/oder das Linsenelement mindestens ein Befestigungselement zum Befestigen des Linsenelements an dem Basiselement aufweisen.

Bei dem zu behandelnden Auge kann es sich beispielsweise um ein Auge eines Patienten handeln, dessen natürliche Linse durch das Linsenimplantat ersetzt werden soll und wobei die natürliche Linse des zu behandelnden Auges bereits aus diesem entfernt wurde. Das Basiselement kann als Basis für das optisch wirksame Linsenelement dienen. Beispielsweise können das Basiselement und das Linsenelement so ausgestaltet sein, dass das Linsenelement auf dem Basiselement befestigt werden kann, während sich das Basiselement bereits in dem zu behandelnden Auge befindet. Das optisch wirksame Linsenelement ist in dem Sinne "optisch wirksam", dass auf dieses Linsenelement eintreffende Lichtstrahlen durch das Linsenelement (beispielsweise durch Brechung) eine Ablenkung erfahren. Bei dem Linsenelement kann es sich beispielsweise im Wesentlichen um eine Sammellinse (positive Linse) handeln. Bei dem Linsenelement kann es sich beispielsweise um eine bi-konvexe, eine plan-konvexe oder um eine konkav-konvexe Linse mit sphärischen oder asphärischen Oberflächen handeln. Die Linse kann beispielsweise eine torische Linse sein, welche zur Korrektur eines Astigmatismus verwendet wird. Das Linsenelement kann beispielsweise bezüglich seiner optischen Achse nicht rotationssymmetrisch ausgestaltet sein. Das Linsenelement kann dazu eingerichtet sein, mittels des mindestens einen Befestigungselements auf dem Basiselement befestigbar zu sein (im Folgenden: befestigter Zustand). Das Basiselement kann so ausgestaltet sein, dass ein Lichtstrahl, welcher im befestigten Zustand das optisch wirksame Linsenelement entlang dessen optischer Achse passiert, von dem Basiselement nicht beeinflusst wird. Bei dem Befestigungselement kann es sich um ein zumindest an dem Basiselement oder dem Linsenelement vorgesehenes beliebiges Element handeln, welches dazu eingerichtet ist, das Linsenelement auf dem Basiselement zu befestigen. Beispielsweise kann entweder das Basiselement oder das Linsenelement, aber auch sowohl das Basiselement als auch das Linsenelement, eines oder mehrere Befestigungselemente aufweisen.

Für das Befestigungselement sind verschiedene Ausgestaltungen denkbar.

Bei dem Befestigungselement kann es sich gemäß einem Beispiel um einen Befestigungsstift handeln, wobei der Befestigungsstift an dem Linsenelement vorgesehen ist und das Basiselement eine Öffnung zum Einführen des Befestigungsstifts aufweist oder wobei der Befestigungsstift an dem Basiselement vorgesehen ist und das Linsenelement eine Öffnung zum Einführen des Befestigungsstifts aufweist.

Der Befestigungsstift kann beispielsweise einen kreisförmigen, ovalen, rechteckigen oder quadratischen Querschnitt aufweisen. Ebenso kann die Öffnung beispielsweise einen kreisförmigen, ovalen, rechteckigen oder quadratischen Querschnitt aufweisen. Der Befestigungsstift und die Öffnung sind so dimensioniert, dass der Befestigungsstift in die Öffnung eingeführt werden kann. In dem befestigten Zustand kann sich ein Abschnitt des Befestigungsstifts in der Öffnung befinden und eine kraftschlüssige Verbindung bilden. In einem nicht-befestigten Zustand kann ein Durchmesser des Befestigungsstifts beispielsweise geringfügig größer sein als ein Durchmesser der Öffnung, so dass der Befestigungsstift beim Einführen in die Öffnung in radialer Richtung komprimiert werden muss. Der Durchmesser des Befestigungsstifts kann jedoch auch im Wesentlichen dem Durchmesser der Öffnung entsprechen oder kleiner sein als der Durchmesser der Öffnung. Das Material des Befestigungsstifts kann beispielsweise dazu eingerichtet sein, nach dem Einführen des Befestigungsstifts in die Öffnung aufzuquellen. Ferner können sowohl das Linsenelement als auch das Basiselement jeweils zumindest einen Befestigungsstift und jeweils zumindest eine Öffnung aufweisen. Die Befestigungsstifte und die zugehörigen Öffnungen können hierbei auf den jeweiligen Elementen so positioniert sein, dass im befestigten Zustand jeweils ein Befestigungsstift in jeweils eine Öffnung eingeführt ist.

Das Linsenelement kann gemäß einem Beispiel zumindest einen Befestigungsstift umfassen und das Basiselement kann zumindest eine Öffnung zum Einführen des Befestigungsstifts und zum Befestigen des Linsenelements an dem Basiselement umfassen.

Das Linsenelement kann gemäß einem Beispiel einen oder mehrere Befestigungsstifte umfassen und das Basiselement kann eine entsprechende Anzahl an Öffnungen umfassen. Die Anordnung der Befestigungsstifte an dem Linsenelement kann der Anordnung der Öffnungen an dem Basiselement entsprechen. Der mindestens eine Befestigungsstift des Linsenelements kann in einem Randbereich des Linsenelements vorgesehen sein. Bei der Öffnung kann es sich beispielsweise um ein Durchgangsloch oder um ein Sackloch handeln. Der mindestens eine Befestigungsstift des Linsenelements kann sich entlang einer optischen Achse des Linsenelements erstrecken.

Der Befestigungsstift kann dazu eingerichtet sein, nach dem Einführen des Befestigungsstifts in die Öffnung des bereits in das zu behandelnde Auge implantierten Basiselements aufzuquellen, um einen Kraftschluss und/oder Formschluss zwischen dem Befestigungsstift und der Öffnung zu erhöhen. Beispielsweise kann ein Material oder eine Materialbeschaffenheit des Befestigungsstifts und/oder des gesamten Linsenelements so gewählt werden, dass der Befestigungsstift nach dem Einführen in die Öffnung zumindest in radiale Richtung aufquillt. Das Aufquellen kann beispielsweise durch eine Temperaturveränderung (Erwärmung) oder durch Befeuchten des Befestigungsstifts ausgelöst werden.

Bei der Öffnung kann es sich um ein Durchgangsloch handeln. Die Länge eines zugehörigen Befestigungsstifts kann so gewählt werden, dass der Befestigungsstift im befestigten Zustand über das Loch hinausragt, bündig mit diesem abschließt, oder das Loch nur teilweise ausfüllt. Wenn es sich bei der Öffnung um ein Durchgangsloch handelt, können ungewollt abgebrochene Befestigungsstifte, welche in dem Durchgangsloch stecken geblieben sind, durch das Durchgangsloch geschoben und somit bequem wieder entfernt werden.

Erfindungsgemäß handelt es sich bei dem Befestigungselement um ein Halteelement. Das Halteelement ist als ösenförmiges Halteelement ausgestaltet. Das ösenförmige Haltelement kann gemäß einem Beispiel an dem Linsenelement vorgesehen sein und das Basiselement kann einen zugehörigen Vorsprung zum Einführen in das ösenförmige Halteelement aufweisen. Erfindungsgemäß ist das ösenförmige Halteelement an dem Basiselement vorgesehen sein und das Linsenelement weist einen zugehörigen Vorsprung zum Einführen in das ösenförmige Halteelement auf.

Das Halteelement ist nicht auf eine bestimmte Form beschränkt. Beispielsweise kann das Halteelement rechteckig, quadratisch, oval, oder kreisförmig sein. Das Halteelement kann eine Ausnehmung oder Öffnung oder Durchführung aufweisen, die sich parallel zu der Oberfläche des Basiselements erstreckt, auf der das Halteelement angeordnet ist. Im Folgenden wird der Einfachheit halber stets von einer Ausnehmung gesprochen. Die Ausnehmung kann auf einer oder zwei Seiten geöffnet sein. In dem Fall, dass die Ausnehmung nur auf einer Seite geöffnet ist, befindet sich die offene Seite der Ausnehmung beispielsweise näher am Zentrum des Basiselements als die nicht offene Seite der Ausnehmung. Die Ausnehmung kann beispielsweise einen rechteckigen, quadratischen, ovalen, oder kreisförmigen Umfang / Querschnitt aufweisen.

Das Basiselement weist erfindungsgemäß eine Vorderseite / Vorderfläche und eine Rückseite / Rückfläche auf. Unter der Vorderseite / Vorderfläche des Basiselements kann beispielsweise die Seite verstanden werden, die nach Einbringen des Basiselements in das Auge näher an der Hornhaut (Cornea) liegt. Dementsprechend kann unter der Rückseite / Rückfläche des Basiselements beispielsweise die Seite verstanden werden, die nach Einbringen des Basiselements in das Auge näher an dem Sehnerv liegt. Die Vorderseite / Vorderfläche und die Rückseite / Rückfläche des Basiselements können prinzipiell die gleiche Form aufweisen.

Auf jeder Seite des Basiselements kann mindestens ein Befestigungselement vorhanden sein. Beispielsweise kann an jeder Seite des Basiselements mindestens ein Halteelement ausgebildet sein. Auf der Vorderseite / Vorderfläche und der Rückseite/ Rückfläche des Basiselements kann eine identische Anzahl an Halteelementen oder eine unterschiedliche Anzahl an Halteelementen ausgebildet sein. Rein beispielhaft sei hier genannt, dass an der Vorderseite / Vorderfläche des Basiselements drei Halteelemente und an der Rückseite / Rückfläche des Basiselements zwei Halteelemente ausgebildet sein können. Beispielsweise kann das mindestens eine ösenförmige Haltelement an der Vorderfläche des Basiselements angeordnet ein und die Rückfläche des Basiselements kann zumindest ein weiteres ösenförmiges Haltelement aufweisen. Auf diese Weise kann ein erstes Linsenelement an der Vorderfläche des Basiselements befestigt werden und kann ein zweites, beispielsweise andersartiges, Linsenelement an der Rückfläche des Basiselements befestigt werden. Die Haltelemente auf der Vorderfläche können in Umfangsrichtung des Basiselements versetzt zu den Halteelementen der Rückfläche angeordnet sein. Dadurch kann eine möglichst geringe Dicke des Basiselements, z.B. von maximal von 0,5 µm, realisiert werden. Dies ermöglicht eine möglichst kleine Inzisionsgröße. Zum Beispiel können die Halteelemente auf der Vorder- und Rückfläche in radialer Richtung jeweils an der gleichen Position angeordnet sein.

In dem befestigten Zustand kann sich zumindest ein Abschnitt des Vorsprungs in dem Halteelement befinden und - je nach betrachteter Richtung - eine kraftschlüssige und/oder formschlüssige Verbindung mit dem Haltelement bilden. Beispielsweise kann das Halteelement eine Bewegung des Vorsprungs in der Ausnehmung nur in eine Richtung gestatten und in allen weiteren Richtungen, beispielsweise auf Grund des zwischen dem Vorsprung und dem Halteelement bestehenden Formschlusses, eine Bewegung blockieren. Bei der Richtung, in welche eine Bewegung gestattet ist, kann es sich um eine radiale Richtung handeln.

In einem nicht-befestigten Zustand kann der Umfang des Vorsprungs beispielsweise geringfügig größer sein als der Umfang der Ausnehmung des Halteelements, so dass der Vorsprung beim Einführen in die Ausnehmung komprimiert werden muss. Der Umfang des Vorsprungs kann jedoch auch im Wesentlichen dem Umfang der Ausnehmung entsprechen oder kleiner sein als der Umfang der Ausnehmung. Das Material des Vorsprungs kann beispielsweise dazu eingerichtet sein, nach dem Einführen des Vorsprungs in die Ausnehmung aufzuquellen. In einem Ausführungsbeispiel kann das Linsenelement zumindest einen Vorsprung umfassen und das Basiselement kann zumindest eine Halteelement zum Einführen des Vorsprungs und zum Befestigen des Linsenelements an dem Basiselement umfassen. Beispielsweise kann ein Material oder eine Materialbeschaffenheit des Vorsprungs und/oder des gesamten Linsenelements so gewählt werden, dass der Vorsprung nach dem Einführen in die Ausnehmung zumindest in eine Richtung aufquillt. Das Aufquellen kann beispielsweise durch eine Temperaturveränderung (Erwärmung) oder durch Befeuchten des Vorsprungs ausgelöst werden. Ferner können sowohl das Linsenelement als auch das Basiselement jeweils zumindest einen Vorsprung und jeweils zumindest eine Ausnehmung aufweisen. Die Vorsprünge und die zugehörigen Ausnehmungen können hierbei auf den jeweiligen Elementen so positioniert sein, dass im befestigten Zustand jeweils ein Vorsprung in jeweils eine Ausnehmung eingeführt ist.

Wie genannt, kann der Vorsprung dazu eingerichtet sein, nach dem Einführen des Vorsprungs in das ösenförmige Haltelement aufzuquellen, um einen Kraftschluss und/oder Formschluss zwischen dem Vorsprung und dem ösenförmigen Haltelement zu erhöhen. Der Vorsprung kann dazu eingerichtet sein, nach dem Einführen des Vorsprung in das ösenförmige Haltelement des bereits in das zu behandelnde Auge implantierten Basiselements aufzuquellen, um einen Kraftschluss und/oder Formschluss zwischen dem Vorsprung und dem ösenförmigen Haltelement zu erhöhen.

Zumindest das Basiselement oder das Linsenelement kann eine Mehrzahl an Befestigungselementen aufweisen. Das Vorsehen mehrerer Befestigungselemente kann es ermöglichen, dass im befestigten Zustand mehrere Verbindungspunkte (Ankerpunkte) zwischen dem Basiselement und dem Linsenelement bestehen. Dies kann die Genauigkeit der Positionierung bzw. Ausrichtung sowie die Rotationsstabilität des Linsenelements in Bezug auf das Basiselement verbessern.

Das Basiselement ist erfindungsgemäß im Wesentlichen plattenförmig. Die Platte erstreckt sich hierbei in einer Ebene oder weist eine Wölbung auf, welche einer Wölbung der Hornhaut oder des Linsensacks des zu behandelnden Auges folgt. Das plattenförmige Basiselement kann beispielsweise im Wesentlichen entlang einer Kugeloberfläche gewölbt sein. Das plattenförmige Linsenelement kann in Aufsicht (Blick entlang einer Normalen der Platte) im Wesentlichen kreisförmig sein und/oder eine kreisförmige Außenkante aufweisen. In dem plattenförmigen Basiselement können Öffnungen (zum Beispiel Durchgangsöffnungen) vorgesehen sein, zum Einführen von Befestigungsstiften des Linsenelements. Das plattenförmige Basiselement kann eine Aussparung aufweisen, durch welche im befestigten Zustand die optische Achse des Linsenelements verläuft. Die Aussparung kann so dimensioniert sein, dass sie im befestigten Zustand in Aufsicht einen Großteil der Fläche des Linsenelements überlappt. Hierdurch kann eine möglichst große Lichtausbeute erreicht werden, so dass ein möglichst großer Teil der auf das zu behandelnde Auge eintreffenden Lichtstrahlen die Netzhaut des zu behandelnden Auges erreichen.

Das Basiselement kann scharfkantige Außenkanten mit einem Kantenwinkel von maximal 90° aufweisen. Ein plattenförmiges Basiselement kann beispielsweise in zumindest einem Bereich seiner Außenkanten so abgeflacht sein, dass eine Oberseite und eine Unterseite des Basiselements unter einem spitzen Winkel zueinander zulaufen und somit eine scharfkantige Außenkante bilden. Durch das Vorsehen scharfer Außenkanten kann eine Nachstarrbildung verhindert werden.

Das Basiselement weist erfindungsgemäß die Form eines Ringsegments auf. Das Basiselement kann hierbei beispielsweise nahezu einen vollständig geschlossenen Ring mit einer Öffnung aufweisen. Die Öffnung des Ringsegments kann beispielsweise weniger als 25% (90°) eines vollständig geschlossenen Rings betragen. Die Öffnung beträgt erfindungsgemäß mehr als 5% (18°) eines vollständig geschlossenen Rings. Die Außenkanten des Ringsegments, welche in Aufsicht zu der Öffnung des Ringsegments weisen, können in Aufsicht abgerundet sein. Das Basiselement kann in Aufsicht im Wesentlichen C-förmig bzw. U-förmig ausgestaltet sein. Ein Basiselement in Form eines Ringsegments hat im Vergleich zu einem ringförmigen Basiselement den Vorteil, dass es durch eine kleinere Inzision in das zu behandelnde Auge implantiert werden kann, wobei eine Inzision einer Länge ausreichend ist, welche im Wesentlichen der Differenz des Außendurchmessers und des Innendurchmessers des Ringsegments entspricht.

Das Basiselement kann mindestens einen Vorsprung aufweisen, welcher sich radial in Richtung eines Mittelpunkts des Ringsegments erstreckt und der Vorsprung kann eine Öffnung zum Einführen eines Befestigungsstifts des Linsenelements aufweisen.

Der Vorsprung kann beispielsweise im Wesentlichen in Form eines Kreissegments ausgestaltet sein. Das Basiselement kann beispielsweise drei Vorsprünge aufweisen, welche im Wesentlichen an den Eckpunkten eines gleichschenkligen (zum Beispiel eines gleichseitigen) Dreiecks angeordnet sind. Jeder der Vorsprünge kann jeweils eine Öffnung aufweisen. Ein Abstand von einer Öffnung zu einer benachbarten Öffnung kann beispielsweise für jede der Öffnungen konstant sein. Im befestigten Zustand können in Aufsicht lediglich der Vorsprung oder die Vorsprünge das Linsenelement überlappen, so dass ein möglichst großer Bereich des Linsenelements nicht von dem Basiselement überlappt wird.

Das Basiselement kann beispielsweise Polymethylmethacrylat, PMMA, und/oder eine lichtundurchlässige Beschichtung aufweisen. Das Basiselement kann beispielsweise im Wesentlichen aus PMMA gebildet sein. Das Basiselement kann entweder teilweise oder vollständig lichtundurchlässig beschichtet sein. Eine lichtundurchlässige Beschichtung kann Streulichteffekte (beispielsweise bei großer Pupille) verhindern und kann beispielsweise optische Phänomene (z.B. Flackern) verhindern, welche durch Reflexionen von Licht innerhalb des Basiselements in einem Randbereich des Basiselements entstehen können.

Das Linsenelement kann im Wesentlichen kreisförmig sein. Ein Durchmesser des kreisförmigen Linsenelements kann beispielsweise im Wesentlichen dem Innendurchmesser des ringsegmentförmigen Basiselements entsprechen oder geringfügig geringer sein als dieser Innendurchmesser. Der Durchmesser des kreisförmigen Linsenelements kann beispielsweise auch größer sein als der Innendurchmesser des ringsegmentförmigen Basiselements. Hierdurch kann durch ein Aufliegen an der Oberfläche des ringsegmentförmigen Basiselements eine zusätzliche Stabilisierung des auf dem Basiselement befestigten Linsenelements erreicht werden. Der Mittelpunkt des kreisförmigen Linsenelements kann im befestigten Zustand in Aufsicht beispielsweise dem Mittelpunkt des ringsegmentförmigen Basiselements entsprechen.

Das Linsenelement kann hydrophobes Acrylat aufweisen. Das Linsenelement kann beispielsweise vollständig aus hydrophoben Acrylat gefertigt sein.

Ein weiterer Aspekt der vorliegenden Offenbarung betrifft ein Basiselement eines Linsenimplantats zum Implantieren in ein zu behandelndes Auge. Das Basiselement umfasst mindestens ein Befestigungselement wie es hierin beschrieben wurde / wird zum Befestigen eines optischen wirksamen Linsenelements wie es hierin beschrieben wurde / wird an dem Basiselement. Beispielsweise kann das Basiselement eine Mehrzahl an Halteelementen oder Öffnungen umfassen wie sie hierin beschrieben wurden / werden. Die Öffnungen können zum Einführen von jeweils einem Befestigungsstift eines optisch wirksamen Linsenelements zum Befestigen des Linsenelements an dem Basiselement ausgebildet sein.

Ein Ausführungsbeispiel eines Basiselements eines Linsenimplantats zum Implantieren in ein zu behandelndes Auge umfasst eine Mehrzahl an ösenförmigen Haltelementen zum Aufnehmen von jeweils einem Vorsprung eines optisch wirksamen Linsenelements zum Befestigen des Linsenelements an dem Basiselement.

Das Basiselement kann hierbei jedes beliebige der zuvor in Bezug auf das Basiselement beschriebenen Merkmale und/oder vorteilhaften Ausgestaltungen aufweisen.

Ein weiterer Aspekt der vorliegenden Offenbarung betrifft ein optisch wirksames Linsenelement eines Linsenimplantats zum Implantieren in ein zu behandelndes Auge. Das Linsenelement ist dazu ausgebildet, an dem Basiselement über das mindestens eine Befestigungselement verbunden zu werden wie dies hierin beschrieben wurde / wird. Beispielsweise kann das Linsenelement eine Mehrzahl an Befestigungsstiften zum Einführen in jeweils eine Öffnung eines Basiselements zum Befestigen des Linsenelements an dem Basiselement aufweisen.

Ein Ausführungsbeispiel eines optisch wirksamen Linsenelements eines Linsenimplantats zum Implantieren in ein zu behandelndes Auge umfasst eine Mehrzahl an Vorsprüngen zum Einführen in jeweils ein ösenförmiges Haltelement eines Basiselements zum Befestigen des Linsenelements an dem Basiselement.

Das Linsenelement kann hierbei jedes beliebige der zuvor in Bezug auf das Linsenelement beschriebenen Merkmale und/oder vorteilhaften Ausgestaltungen aufweisen.

### Kurze Beschreibung der Zeichnungen

Ergänzende Merkmale, Vorteile und Bestandteile der vorliegenden Erfindung sind aus der nachfolgenden Beschreibung der beigefügten Zeichnungen zu entnehmen, in welchen:
- Figur 1a: eine schematische Aufsicht eines ersten nicht erfindungsgemäßen Beispiels eines Basiselements zeigt;
- Figur 1b: einen Querschnitt des ersten Beispiels des Basiselements der Figur 1a entlang der Linie A-A zeigt;
- Figur 2a: eine schematische Aufsicht eines ersten nicht erfindungsgemäßen Beispiels eines Linsenelements zeigt;
- Figur 2b: einen Querschnitt des ersten Beispiels des Linsenelements der Figur 2a entlang der Linie B-B zeigt;
- Figur 3: eine schematische Aufsicht eines erfindungsgemäßen Ausführungsbeispiels eines Basiselements zeigt;
- Figur 4a: eine schematische Untersicht des Ausführungsbeispiels des Basiselements aus Figur 3 zeigt;
- Figuren 4b bis 4e: verschiedene Schnitte und Seitenansichten des Ausführungsbeispiels des Basiselements der Figur 4a zeigen;
- Figur 5: eine schematische Aufsicht eines erfindungsgemäßen Ausführungsbeispiels eines Linsenelements zeigt;
- Figur 6: eine schematische Aufsicht eines erfindungsgemäßen Ausführungsbeispiels eines weiteren Linsenelements zeigt; und
- Figur 7: eine schematische perspektivische Ansicht des Basiselements aus Figur 3, des Linsenelements aus Figur 5 und des weiteren Linsenelements aus Figur 6 zeigt.

### Detaillierte Beschreibung beispielhafter Ausführungsformen

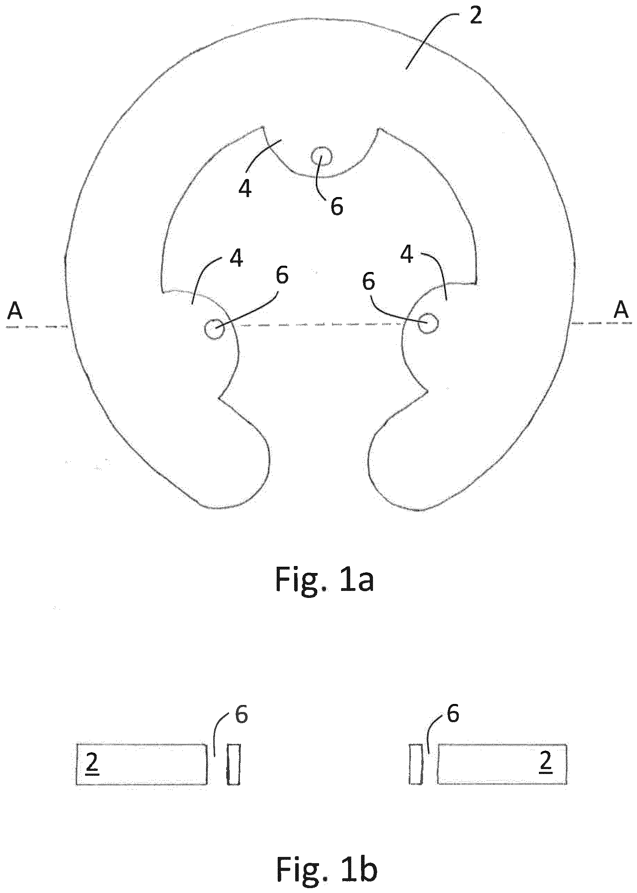

Die Figur 1a zeigt ein erstes Beispiel eines Basiselements 2 in Aufsicht. Das Basiselement 2 ist hierbei plattenförmig, d.h. eine Erstreckung des Basiselements 2 in die Richtung senkrecht zur Zeichenebene ist deutlich geringer als die Erstreckung des Basiselements 2 innerhalb der in Figur 1a dargestellten Zeichenebene. Die Aufsicht der Figur 1a entspricht somit einem Blick auf das Basiselement 2 entlang einer Flächennormalen des plattenförmigen Basiselements 2. Die Figur 1b zeigt einen Querschnitt des in der Figur 1a dargestellten Basiselements 2 entlang der Linie A-A. Sämtliche der beigefügten Figuren dienen lediglich der Veranschaulichung und die darin dargestellten Elemente sind nicht zwangsläufig maßstabsgetreu abgebildet und können auf vielfältige Weise unterschiedlich dimensioniert sein. Ferner handelt es sich bei den Figuren lediglich um beispielhafte Realisierungen, d.h. nicht jedes der dort gezeigten und im Folgenden beschriebenen Elemente muss tatsächlich vorhanden sein.

Wie in der Figur 1b ersichtlich ist, weist das dargestellte Basiselement 2 keine Krümmung auf und erstreckt sich im Wesentlichen innerhalb der Zeichenebene der Figur 1a. Es sind jedoch auch andere Ausführungsformen möglich, in denen das plattenförmige Basiselement 2 beispielsweise im Wesentlichen entlang einer Zylinderoberfläche oder einer Kugeloberfläche gekrümmt ist. Die Außenkanten des Basiselements 2 sind im Querschnitt im Wesentlichen rechtwinklig, können jedoch in anderen Ausführungsformen auch abgerundet sein oder beispielsweise so zugespitzt sein, dass die Oberseite und die Unterseite des plattenförmigen Basiselements 2 an ihren Seitenrändern nicht parallel sind, sondern aufeinander zulaufen und somit an den Seitenrändern einen spitzen Winkel bilden.

Das Basiselement 2 ist in Aufsicht im Wesentlichen C-förmig und weist somit im Wesentlichen die Form eines Kreisringsegments bzw. Ringsegments auf. In einer anderen Ausführungsform ist es jedoch auch möglich, dass das Basiselement 2 in Aufsicht die Form eines geschlossenen Kreisrings aufweist. Dies kann zu einer erhöhten Stabilität des Basiselements 2 führen.

Das ringsegmentförmige Basiselement 2 weist an einer Stelle eine Öffnung des Ringsegments auf, welche in der Darstellung der Figur 1a nach unten gerichtet ist. Die Außenkanten des Basiselements 2, welche zu dieser Öffnung des Ringsegments hindeuten, sind in Aufsicht abgerundet und beschreiben die Form eines Halbkreises. Hierdurch lässt sich das Basiselement 2 einfach in eine Inzision des zu behandelnden Auges einführen, wodurch Verletzungen vermieden werden können.

Das Basiselement 2 weist ferner drei Vorsprünge 4 auf, welche sich in Aufsicht ausgehend von der Form des Ringsegments in radiale Richtung (zu dem Mittelpunkt des Ringsegments hin) erstrecken. Die Vorsprünge 4 weisen hierbei im Wesentlichen jeweils die Form eines Kreissegments auf. In jedem der Vorsprünge 4 ist eine Öffnung 6 vorgesehen. Die Öffnungen 6 durchdringen das plattenförmige Basiselement 2 in eine Richtung entlang einer Flächennormale des plattenförmigen Basiselements 2, d.h. senkrecht zur Zeichenebene der Figur 1a. Bei den Öffnungen 6 handelt es sich um durchgehende Löcher. In anderen Beispielen können jedoch als Öffnungen 6 auch beispielsweise Sacklöcher vorgesehen sein. Die Öffnungen 6 des Basiselements 2 sind so angeordnet, dass sie sich an den Eckpunkten eines gleichschenkligen (zum Beispiel eines gleichseitigen) Dreiecks befinden. Hierbei liegt der Mittelpunkt des Ringsegments innerhalb des Dreiecks und beispielsweise auf einem Mittelpunkt des Dreiecks.

Das Basiselement 2 ist im Wesentlichen aus Polymethylmethacrylat (PMMA) gebildet. PMMA als Basismaterial hat die Vorteile, dass es durchsichtig ist, verhältnismäßig fest und stabil ist und keine Abstoßungs- oder Unverträglichkeitsreaktionen durch das menschliche Auge bekannt sind. Der Außendurchmesser des ringsegmentförmigen Basiselements 2 liegt beispielsweise im Bereich zwischen 11 und 14 mm. Hierbei kann es denkbar sein, den Außendurchmesser des Basiselements 2 an die individuelle Kapselsackgröße des zu behandelnden Auges anzupassen. Somit kann eine individuelle Anpassung des Basiselements 2 an das zu behandelnde Auge des Patienten gewährleistet werden. Der Innendurchmesser des Kreisrings kann beispielsweise 8 mm betragen. Die Dimensionierung des Innendurchmessers wird hierbei zum Beispiel so gewählt, dass eine Sichtbarkeit der Öffnungen 6 während der Operation gewährleistet ist. Die Dicke des Basiselements 2 (siehe Querschnitt der Figur 1b) beträgt beispielsweise 1 mm. Hierbei ist es möglich, die Dicke einerseits so groß zu wählen, dass die Stabilität des Basiselements 2 gewährleistet bleibt, sie jedoch so gering zu wählen, dass das Basiselement 2 sich leicht durch eine Inzision implantieren lässt.

Die C-förmige bzw. ringsegmentförmige Form des Basiselements 2 ermöglicht ein Implantieren des Basiselements 2 in die Vorderkammer bzw. in den Kapselsack des zu behandelnden Auges durch eine kleine Inzision. Die Länge der notwendigen Inzision entspricht im Wesentlichen der Breite (Außendurchmesser minus Innendurchmesser) des Basiselements 2 plus der Länge der Erstreckung des Vorsprungs 4 in radiale Richtung. Weist das Basiselement 2 hingegen im Wesentlichen die Form eines geschlossenen Rings auf, so muss die Inzision zumindest so lang sein wie der Außendurchmesser des Basiselements 2. Zum Implantieren des in Figur 1a dargestellten Basiselements 2 ist beispielsweise eine Inzision von ca. 2,5 mm ausreichend.

Die Öffnungen 6 sind unter einem definierten Abstand zueinander an den Eckpunkten eines Dreiecks (beispielsweise eines gleichschenkligen oder gleichseitigen Dreiecks) angeordnet. Hierdurch wird eine Rotationsstabilität des später beschriebenen Linsenelements 8 bezüglich des Basiselements 2 gewährleistet, wenn das Linsenelement 8 auf dem Basiselement 2 befestigt ist (befestigter Zustand des Linsenimplantats). Bei den Öffnungen 6 des Basiselements 2 handelt es sich um Durchgangslöcher, welche das plattenförmige Basiselement 2 von einer Vorderfläche bis zu einer Rückfläche des Basiselements 2 durchdringen. Falls bei einem Austausch oder beim Einsetzen des Linsenelements 8 ein Befestigungsstift 10 des Linsenelements 8 abbricht und innerhalb einer der Öffnungen 6 stecken bleibt, kann dieser abgebrochene Befestigungsstift 10 durch die Durchgangsöffnung 6 geschoben werden und aus dem zu behandelnden Auge einfach entfernt werden. Die Öffnungen 6 des Basiselements 2 verlaufen entlang einer Flächennormale des plattenförmigen Basiselements 2. Somit wirken beim Einführen der Befestigungsstifte 10 des Linsenelements 8 in die Öffnungen 6 des Basiselements 2 lediglich Kräfte entlang dieser Flächennormale und keine Rotationskräfte innerhalb der Ebene des Basiselements 2, welche zu einer Verdrehung (Rotation) des Basiselements 2 führen könnten. Hierdurch kann eine Rotationsstabilität des Linsenelements 8 in Bezug auf das zu behandelnde Auge gewährleistet werden.

Eine Rückfläche des Basiselements 2 kann eine lichtundurchlässige Beschichtung aufweisen. Hierdurch können Streulichteffekte bei großen Pupillen verhindert werden, welche z.B. durch Licht verursacht werden, das an den Öffnungen 6 bzw. den Befestigungsstiften 10 gestreut wird. Ränder des Basiselements 2 können eine lichtundurchlässige Beschichtung aufweisen. Hierdurch können optische Phänomene (z.B. Flackern) verhindert werden, welche durch Fortleitung von Licht (beispielsweise Totalreflektion und anschließende Auskopplung) innerhalb des Basiselements 2 am Rand des Basiselements 2 entstehen können. Das Basiselement 2 kann ferner scharfe Kanten aufweisen, wodurch eine Nachstarrbildung verhindert werden kann.

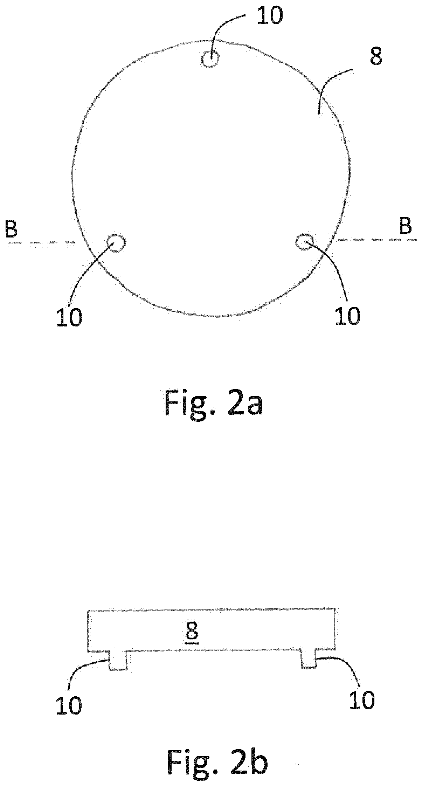

Figur 2a zeigt ein zweites Beispiels eines Linsenelements 8 in Aufsicht und Figur 2b zeigt einen Querschnitt des Linsenelements 8 der Figur 2a entlang der Linie B-B. Das Linsenelement 8 der Figur 2a ist dazu eingerichtet bzw. so dimensioniert, dass es an dem Basiselement 2 der Figur 1a befestigt werden kann. Hierzu weist das Linsenelement 8 drei Befestigungsstifte 10 auf, deren Anordnung in Aufsicht der Anordnung der Öffnungen 6 des Basiselements 2 entspricht. Die Befestigungsstifte 10 des Linsenelements 8 können jeweils in eine Öffnung 6 des Basiselements 2 eingeführt werden. Hierbei wird das Linsenelement 8 in einer Richtung senkrecht zu den Zeichenebenen der Figur 1a bzw. der Figur 2a auf das Basiselement 2 aufgesetzt und befestigt. Hinsichtlich der in den Figuren 1b und 2b dargestellten Querschnitten bedeutet dies, dass die Befestigungsstifte 10 des Linsenelements 8 von oben in die Öffnungen 6 des Basiselements 2 eingeführt werden. Hierbei ist ein Durchmesser der Befestigungsstifte so gewählt, dass diese in die Öffnungen eingesetzt werden können, ohne übermäßig viel Kraft aufwenden zu müssen, aber auch so, dass die Befestigungsstifte 10 nach der erfolgten Operation kraftschlüssig in den Öffnungen 6 des Basiselements 2 sitzen.

Bei dem Linsenelement 8 handelt es sich um ein optisch wirksames Linsenelement 8, was bedeutet, dass Lichtstrahlen, welche das Linsenelement 8 passieren, eine definierte Ablenkung erfahren. Hierzu ist das Linsenelement 8 aus einem transparenten Material gefertigt und weist zumindest eine optisch wirksame Oberfläche auf. Das Linsenelement 8 kann hierbei individuell an die Bedürfnisse des zu behandelnden Auges angepasst sein, so dass Sehfehler und/oder Aberrationen höherer Ordnung der Hornhaut des zu behandelnden Auges ausgeglichen werden können. Beispielsweise kann es sich bei dem Linsenelement 8 im Wesentlichen um eine Sammellinse handeln, deren optische Eigenschaften im Wesentlichen rotationssymmetrisch bezüglich der optischen Achse des Linsenelements 8 sind.

Das Linsenelement 8 kann jedoch auch nicht rotationssymmetrisch bezüglich seiner optischen Achse ausgebildet sein, insbesondere wenn ein Astigmatismus des zu behandelnden Auges ausgeglichen werden soll. Hierbei muss auf eine exakte Rotationsausrichtung des Linsenelements 8 in Bezug auf das zu behandelnde Auge geachtet werden. Eine entsprechende Rotationsausrichtung bzw. Rotationsstabilität kann durch das Vorsehen der drei Befestigungsstifte gewährleistet werden. Das Linsenelement 8 kann beispielsweise im Wesentlichen eine torische Linse darstellen. Bei dem Linsenelement 8 kann es sich beispielsweise um eine plan-konvexe Linse handeln, bei welcher die Unterseite des Linsenelements 8, welche im befestigten Zustand dem Basiselement 2 zugewandt ist, planar ist, und bei welcher eine optisch aktive Strukturierung auf der der Unterseite gegenüberliegenden Oberseite des Linsenelements 8 vorgenommen wurde. Es ist zu beachten, dass es sich bei dem Querschnitt der Figur 2b lediglich um eine schematische Darstellung handelt, welche keine entsprechende optisch aktive Strukturierung der Oberflächen des Linsenelements 8 zeigt. Bei dem dargestellten Linsenelement kann es sich beispielsweise um einen Rohling handeln, welcher noch nicht eingeschliffen wurde. Das Linsenelement 8 kann jedoch hinsichtlich seiner optischen Eigenschaften beliebig an die Bedürfnisse des zu behandelnden Auges angepasst werden.

Das Linsenelement 8 der Figur 2a ist in Aufsicht kreisrund, wobei die optische Achse des Linsenelements 8 durch einen Mittelpunkt des Kreises und entlang einer Flächennormale der Zeichenebene der Figur 2a verläuft. Somit kann das Linsenelement 8 als Austausch für die natürliche Linse des zu behandelnden Auges dienen. Der Durchmesser des Linsenelements 8 kann beispielsweise 7 mm betragen. Das Linsenelement 8 ist aus hydrophoben Acrylat geformt. Dies hat den Vorteil, dass das Linsenelement 8 faltbar ist, leicht über ein Injektorsystem in das zu behandelnde Auge implantierbar ist, durchsichtig ist, auf einer Drehbank formbar ist, keine Einschlüsse von Luftbläschen aufweist, und das keine Abstoßungen oder Unverträglichkeiten des menschlichen Auges bekannt sind.

Die Länge der Befestigungsstifte 8 kann beispielsweise 0,75 mm betragen. Die Befestigungsstifte 10 können aus einem Material bestehen, welches nach dem Einsetzen in die Öffnungen 6 des Basiselements 2 aufquillt, wenn sich das Basiselement 2 bereits in dem zu behandelnden Auge befindet. Die Quellung kann beispielsweise innerhalb von drei Minuten geschehen, so dass ein Kraftschluss zwischen dem Befestigungsstiften 10 und den Öffnungen 6 im befestigten Zustand hergestellt bzw. erhöht wird. Beim Befestigen wird durch nur leichtes Andrücken des Linsenelements 8 auf das Basiselement 2 keine tangentiale Kraft auf die hintere Linsenkapsel und somit auf den Zonulaapparat ausgeübt. Bei einem Wechsel des Linsenelements 8 ist die Verbindung leicht wieder lösbar.

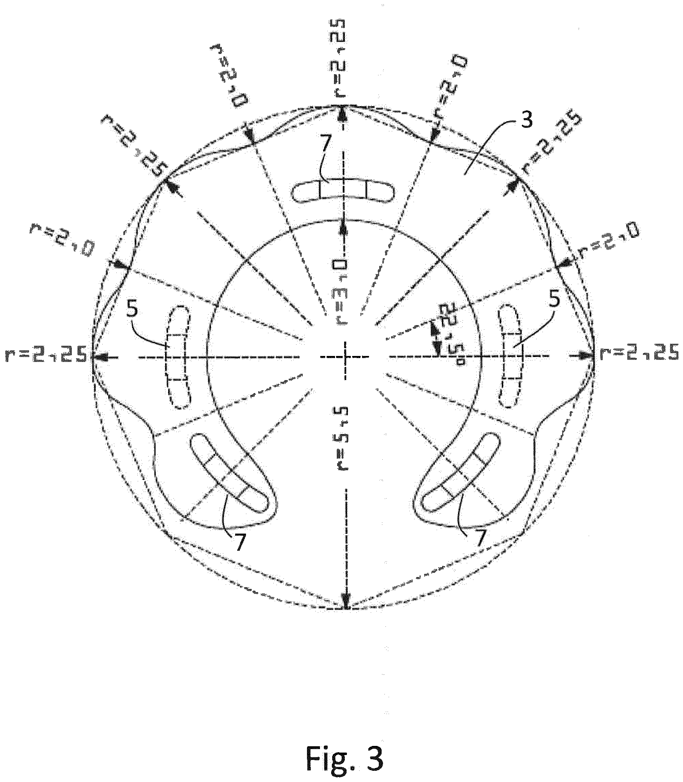

Die Figur 3 zeigt ein erfindungsgemäßes Ausführungsbeispiel eines Basiselements 3 in Aufsicht. Im Folgenden werden der Kürze halber im Wesentlichen die Unterschiede zu dem Basiselement 2 gemäß dem ersten Beispiel erläutert. Die in Bezug auf die Figuren 1a und 1b erläuterten Details können, müssen jedoch nicht, auf ähnliche Weise in dem Basiselement 3 gemäß dem Ausführungsbeispiel realisiert sein.

Wie in Figur 3 zu erkennen, weist das Basiselement 3 im Gegensatz zu dem Basiselement 2 keine Öffnungen 6 auf, die das Basiselement senkrecht, d.h. entlang einer Flächennormale der Zeichenebene aus Figur 3, durchdringen. Hingegen sind in dem Basiselement 3 ösenförmige Halteelemente vorgesehen, die im Folgenden der Kürze halber als Ösenelemente bezeichnet werden. Zudem sind im Gegensatz zu dem Basiselement 2 die Ösenelemente auf unterschiedlichen Seiten, nämlich der Vorder- und der Rückseite, des Basiselements 3 angeordnet. Rein beispielhaft sind drei Ösenelemente 7 auf der Vorderfläche des Basiselements 3 vorgesehen, während zwei Ösenelemente 5 auf der Rückfläche des Basiselements 3 vorgesehen sind. Die Ösenelemente 7 auf der Vorderfläche sind in Umfangsrichtung des Basiselements 3 versetzt zu den Ösenelementen 5 der Rückfläche angeordnet, in radialer Richtung beispielhaft jedoch jeweils an der gleichen Position. Dadurch kann eine möglichst geringe Dicke des Basiselements realisiert werden. Beispielsweise kann erreicht werden, dass beim Implantieren eine maximale Dicke von 0,5 µm an der breitesten Stelle nicht überschritten wird und somit eine möglichst kleine Inzisionsgröße ermöglicht wird.

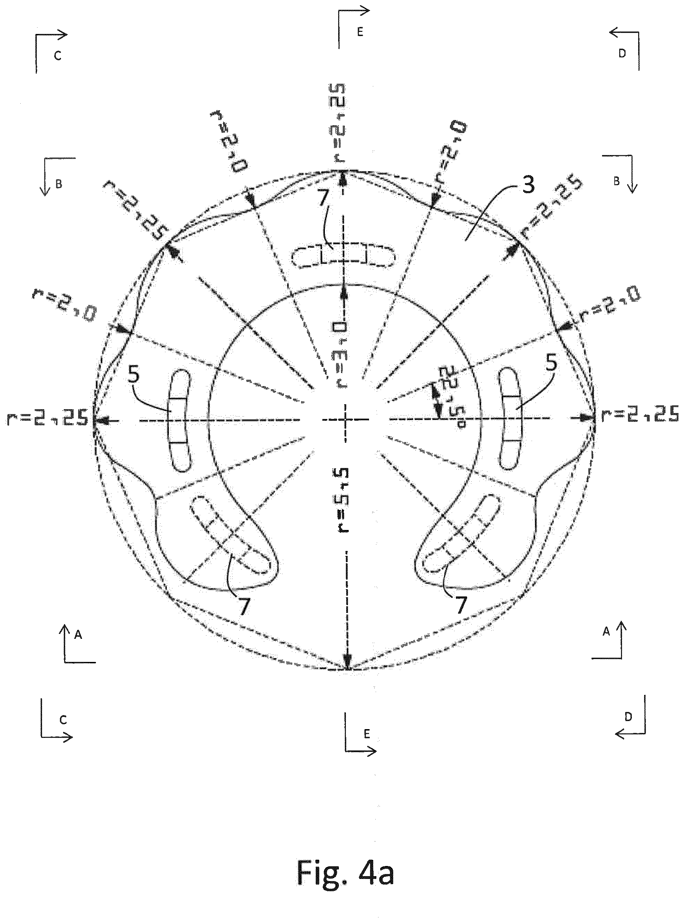

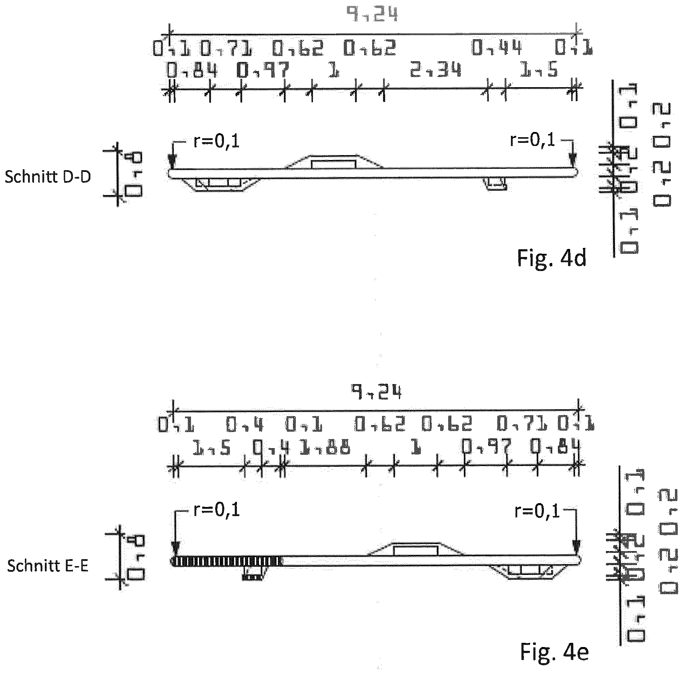

Eine Untersicht auf das Basiselement 3 ist in Figur 4a gezeigt. Dementsprechend zeigt die Figur 4a eine Aufsicht auf die Rückfläche des Basiselements 3.

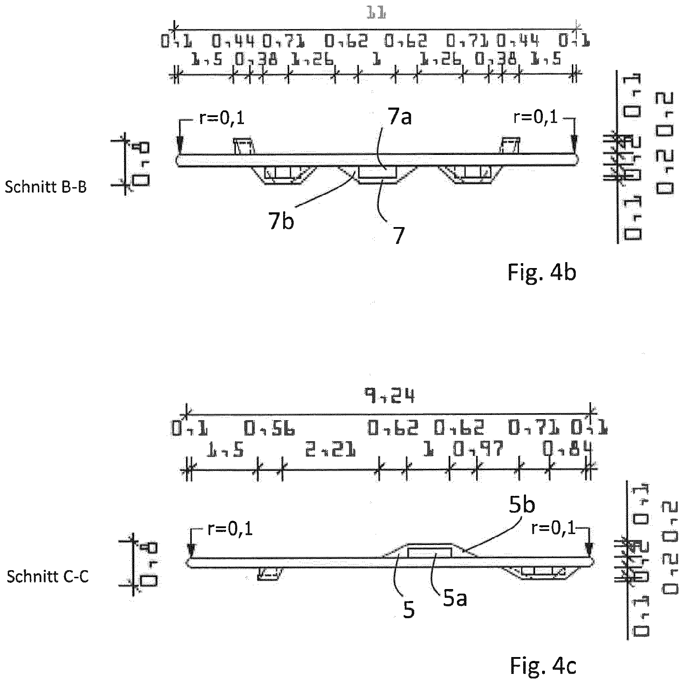

Die Ösenelemente 5, 7 sind jeweils so geformt, dass sich deren Ausnehmungen 5a, 7a in radialer Richtung des Basiselements 3 erstrecken. Die Ösenelemente ragen jeweils gegenüber der Vorder- und Rückfläche des Basiselements 3 vor. Weitere Details sind aus in den Figuren 4b bis 4e dargestellten Schnitten und Seitenansichten des Basiselements 3 zu erkennen. Der Kürze halber werden die Schnitte und Seitenansichten lediglich als Schnitte bezeichnet und werden nur in Bezug auf die Seitenansichten B-B und C-C aus Figuren 4b und 4c näher erläutert.

Rein beispielhaft wird hierbei angenommen, dass alle Ösenelemente 5, 7 gleichartig aufgebaut sind.

Wie in den Figuren 4b und 4c zu erkennen, weist jedes Ösenelement 5, 7 an seinen Seiten jeweils zwei ansteigende Flanken 5b, 7b auf. In den Ösenelementen 5, 7 ist jeweils eine Ausnehmung 5a, 7a gebildet. Die Ausnehmungen 5a, 7a erstrecken sich jeweils in radialer Richtung des Basiselements 3. Die ansteigenden Flanken 5b, 7b bewirken, dass die Ösenelemente 5, 7 gegenüber dem Basiselement 3 vorstehen.

Da sowohl auf der Vorderfläche als auch auf der Rückfläche des Basiselements 3 Ösenelemente 5, 7 vorgesehen sind, lassen sich zwei Linsenelemente mit dem Basiselement 3 verbinden. Genauer gesagt lässt sich ein Linsenelement an der Vorderfläche und ein Linsenelement an der Rückfläche des Basiselements 3 befestigen.

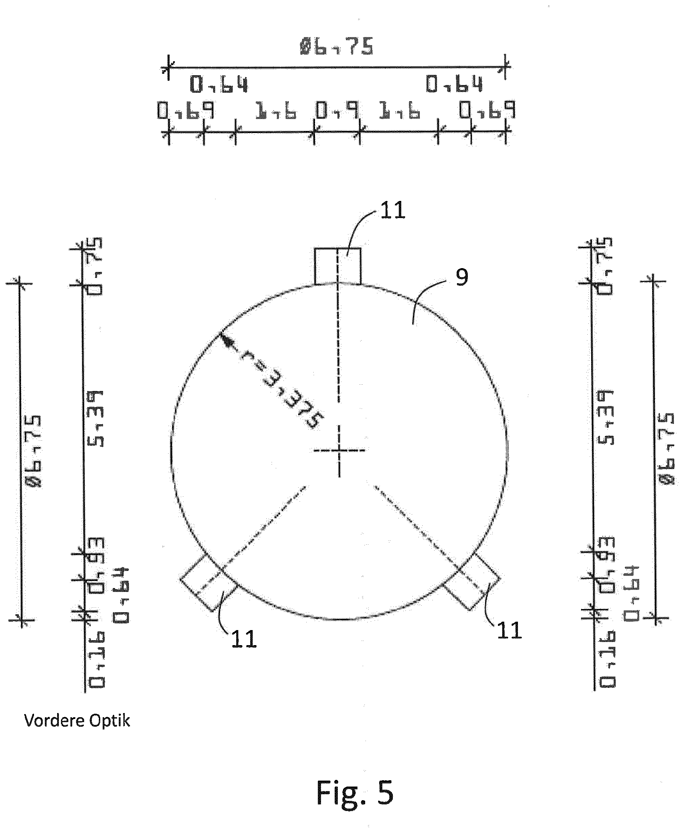

Figur 5 zeigt ein erfindungsgemäßes Ausführungsbeispiel eines Linsenelements 9 in Aufsicht. Im Folgenden werden der Kürze halber im Wesentlichen die Unterschiede zu dem Linsenelement 8 gemäß dem ersten Beispiel erläutert. Die in Bezug auf die Figuren 2a und 2b erläuterten Details können, müssen jedoch nicht, auf ähnliche Weise in dem Linsenelement 9 gemäß dem Ausführungsbeispiel realisiert sein.

Bei dem Linsenelement 9 handelt es sich ebenso wie bei dem Linsenelement 8 um ein optisch wirksames Linsenelement 9, das individuell an die Bedürfnisse des zu behandelnden Auges angepasst sein kann, so dass Sehfehler und/oder Aberrationen höherer Ordnung der Hornhaut des zu behandelnden Auges ausgeglichen werden können.

Das Linsenelement 9 weist im Gegensatz zu dem Linsenelement 8 keine Befestigungsstifte 10 auf, die senkrecht zu der Oberfläche des Linsenelements 8 vorstehen. Stattdessen weist das Linsenelement 3 Vorsprünge 11 auf, die sich radial nach außen erstrecken und in radialer Richtung von dem Linsenelement 9 vorstehen. Die Vorsprünge 11 sind dabei in ihrer Anzahl und Form auf die Ösenelemente 7 und deren Ausnehmungen 7a angepasst. Rein beispielhaft sind daher drei Vorsprünge 11 dargestellt. Dadurch kann das Linsenelement 9 mit der Vorderfläche des Basiselements 3 befestigt werden. Hierfür kann jeder der Vorsprünge 11 radial in seine zugehörige Ausnehmung 7a eingeführt werden. Die Ausnehmungen 7a und die Vorsprünge 11 sind dabei so aufeinander abgestimmt, dass die Ausnehmungen 7a jeweils eine Bewegung des zugehörigen Vorsprungs 11 in radialer Richtung des Basiselements 3 gestatten. In alle weiteren Richtungen wird eine Bewegung des Vorsprungs 11 durch das entsprechende Ösenelement 7 verhindert, da hier ein Formschluss besteht. Die Vorsprünge 11 können aus einem Material bestehen, welches nach dem Einführen in die Ausnehmungen 7a der Ösenelemente 7 des Basiselements 3 aufquillt, beispielsweise wenn sich das Basiselement 3 bereits in dem zu behandelnden Auge befindet. Die Quellung kann beispielsweise innerhalb von drei Minuten geschehen, so dass, z.B. in radialer Richtung, ein Kraftschluss zwischen den Vorsprüngen 11 und den Ösenelementen 7 im befestigten Zustand hergestellt bzw. erhöht wird.

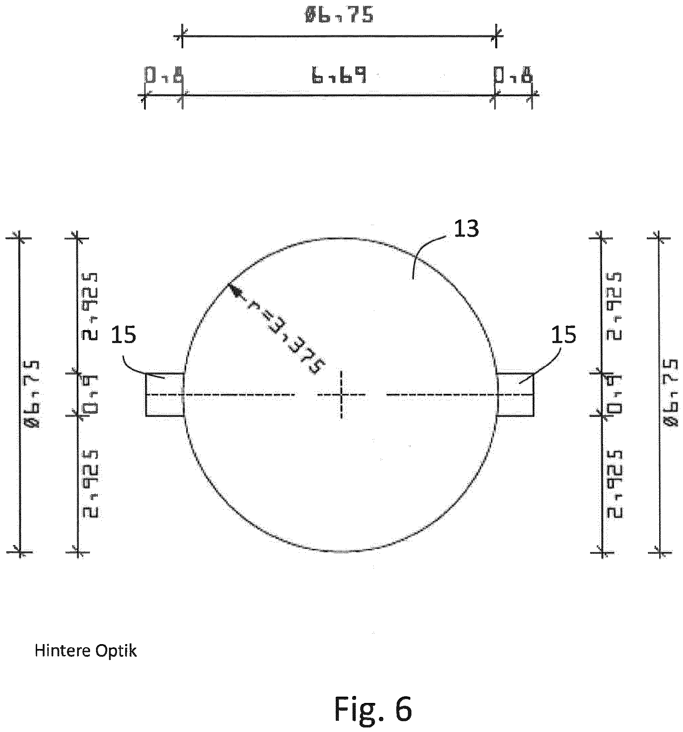

Figur 6 zeigt ein Ausführungsbeispiel eines weiteren Linsenelements 13 in Aufsicht.

Bei dem Linsenelement 13 handelt es sich ebenso wie bei dem Linsenelement 9 um ein optisch wirksames Linsenelement 9. Allerdings ist das Linsenelement 13 nicht individuell an die Bedürfnisse des zu behandelnden Auges angepasst, sondern es handelt sich um eine sphärische Standardlinse zum Ausgleichen von Hyperopie und Myopie.

Das Linsenelement 13 weist im Gegensatz zu dem Linsenelement 9 beispielhaft nur zwei radial nach außen vorstehende Vorsprünge 15 auf. Die Vorsprünge 15 sind dabei in ihrer Anzahl und Form auf die Ösenelemente 5 und deren Ausnehmungen 5a angepasst. Dadurch kann das Linsenelement 13 mit der Rückfläche des Basiselements 3 befestigt werden. Hierfür kann jeder der Vorsprünge 15 radial in seine zugehörige Ausnehmung 5a eingeführt werden. Die Ausnehmungen 5a und die Vorsprünge 15 sind dabei so aufeinander abgestimmt, dass die Ausnehmungen 5a jeweils eine Bewegung des zugehörigen Vorsprungs 15 in radialer Richtung des Basiselements 3 gestatten. In alle weiteren Richtungen wird eine Bewegung des Vorsprungs 15 durch das entsprechende Ösenelement 5 verhindert, da hier ein Formschluss besteht. Die Vorsprünge 15 können aus einem Material bestehen, welches nach dem Einführen in die Ausnehmungen 5a der Ösenelemente 5 des Basiselements 3 aufquillt, beispielsweise wenn sich das Basiselement 3 bereits in dem zu behandelnden Auge befindet. Die Quellung kann beispielsweise innerhalb von drei Minuten geschehen, so dass, z.B. in radialer Richtung, ein Kraftschluss zwischen den Vorsprüngen 15 und den Ösenelementen 5 im befestigten Zustand hergestellt bzw. erhöht wird.

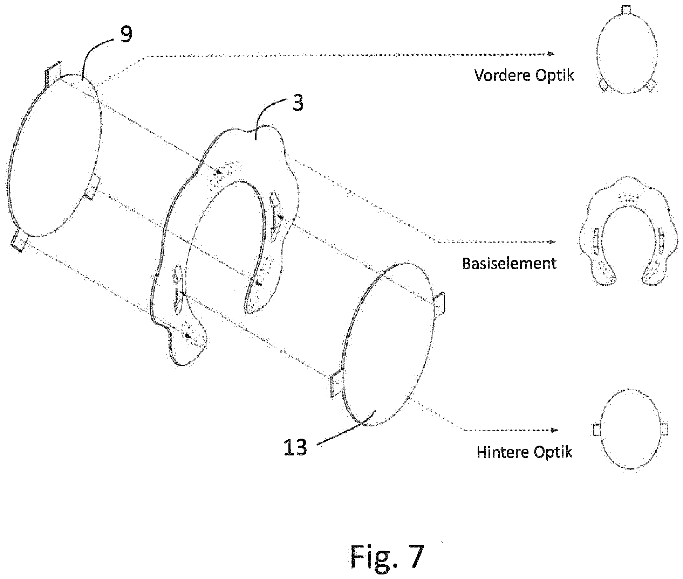

Figur 7 zeigt eine perspektivische Ansicht des Gesamtsystems aus dem Basiselement 3 aus Figuren 3 und 4a, dem Linsenelement 9 aus Figur 5 und dem weiteren Linsenelement 13 aus Figur 6.

Wie vorstehend erläutert, weist das individuell an die Bedürfnisse des zu behandelnden Auges angepasste Linsenelement 9 beispielhaft drei Vorsprünge 11 auf. Da die Anzahl der auf der Vorderfläche des Basiselements 3 angeordneten Ösenelemente 7 auf die Anzahl der Vorsprünge 11 des Linsenelements 9 angepasst ist, und umgekehrt, d.h. die Anzahl der auf der Vorderfläche des Basiselements 3 angeordneten Ösenelemente 7 beispielhaft drei beträgt, kann durch Einbringen der Vorsprünge 11 in die jeweiligen Ösenelemente 7 das Linsenelement 9 an der Vorderfläche des Basiselements 3 befestigt werden. Dementsprechend wird das Linsenelement 9 in der Figur 7 als vordere Optik bezeichnet.

Wie ferner vorstehend erläutert, weist das nicht individuell an die Bedürfnisse des zu behandelnden Auges angepasste Linsenelement 13 (Standardlinse) beispielhaft zwei Vorsprünge 15 auf. Da die Anzahl der auf der Rückfläche des Basiselements 3 angeordneten Ösenelemente 5 auf die Anzahl der Vorsprünge 15 des Linsenelements 13 angepasst ist, und umgekehrt, d.h. die Anzahl der auf der Rückfläche des Basiselements 3 angeordneten Ösenelemente 5 beispielhaft zwei beträgt, kann durch Einbringen der Vorsprünge 15 in die jeweiligen Ösenelemente 5 das Linsenelement 13 an der Rückfläche des Basiselements 3 befestigt werden. Dementsprechend wird das Linsenelement 13 in der Figur 7 als hintere Optik bezeichnet.

Im Folgenden wird ein Verfahren beschrieben, wie das Linsenimplantat bestehend aus dem Basiselement 2, 3 und dem Linsenelement 8, 9 in das zu behandelnde Auge implantiert werden kann. Optional kann ferner das Linsenelement 13 verwendet werden. Zur Ausführung des Verfahrens müssen nicht alle nachfolgend beschriebenen Details durchgeführt werden, d.h. manche Details können auch weggelassen werden.

In einem ersten Schritt wird das Basiselement 2, 3 in die Linsenkapsel des zu behandelnden Auges eingebracht. Anschließend wird die Kapselsackschrumpfung abgewartet, welche ca. drei Monate dauert. In diesem Zustand (nach der Kapselsackschrumpfung) ist das Basiselement 2, 3 stabil in dem zu behandelnden Auge verankert und insbesondere eine Position der Öffnungen 6 bzw. der Ösenelemente 5, 7 in Relation zu den anderen optischen Elementen des zu behandelnden Auges ist festgelegt. Somit kann eine Rotationsstabilität des Basiselements 2, 3 in Bezug auf das zu behandelnde Auge gewährleistet werden.

Falls das Linsenelement 13 verwendet werden soll, wird nach dem ersten Schritt das Linsenelement 13 hinter das Basiselement 2, 3 in die Linsenkapsel implantiert. Hierfür wird, wie beschrieben, das Linsenelement 13 mit Hilfe seiner zwei Vorsprünge 15 an den zwei Ösenelementen 5 der Rückfläche des Basiselements 3 befestigt. Das Linsenelement 13 liegt dann an der hinteren Kapsel an und verfügt über eine 360° scharfe Kante zur Verhinderung eines Nachstars. Mit Hilfe des Linsenelements 13 wird erreicht, dass im Falle des Auftretens eines Nachstars eine Kapseleröffnung mit einem YAG-Laser durchgeführt werden kann, ohne dass der Glaskörper des Auges vom hinteren Augensegment in das vordere Augensegment kommen kann. Das Linsenelement 13 stellt sozusagen eine Barriere dar.

Im Anschluss kann für eine gewisse Zeit die Heilung abgewartet werden.

In einem zweiten Schritt wird die Position der Öffnungen 6 des Basiselements 2 bzw. des Ösenelements 7 des Basiselements 3 in Bezug auf das zu behandelnde Auge festgestellt. Dies kann beispielsweise mit Hilfe bekannter optischer Methoden (beispielsweise mit Hilfe eines Mikroskops) durchgeführt werden.

Ferner wird eine Analyse des zu behandelnden Auges durchgeführt. Die Analyse dient dazu, ein gewünschtes Oberflächenprofil des Linsenelements 8, 9 zu bestimmen. Hierbei soll das Linsenelement 8, 9 eine möglichst scharfe Abbildung wahrgenommener Objekte auf der Netzhaut gewährleisten. Bei der Analyse kann es sich beispielsweise um eine Wellenfront-Analyse und/oder um eine Ermittlung der Topographie der Hornhaut handeln. Die Analyse kann entweder nach dem zweiten Schritt oder bereits zuvor durchgeführt werden. Mit Hilfe der Analyse kann beispielsweise eine Messung von Restfehlsichtigkeiten niedriger und höherer Ordnung durchgeführt werden.

In einem dritten Schritt wird basierend auf der Analyse ein Oberflächenprofil des Linsenelements 8, 9 festgelegt und das Linsenelement 8, 9 entsprechend angefertigt (beispielsweise eingeschliffen).

In einem vierten Schritt wird das Linsenelement 8, 9 an dem Basiselement 2, 3 befestigt, welches sich bereits rotationsstabil in dem zu behandelnden Auge befindet.

Somit kann eine Rotationsstabilität des Linsenelements 8, 9 in Bezug auf das zu behandelnde Auge gewährleistet werden und eine präzise Positionierung des individuell angefertigten Linsenelements 8, 9 in Bezug auf das zu behandelnde Auge ist möglich.

Es ist zu beachten, dass es sich bei dem zuvor beschriebenen Basiselement 2, 3 und dem Linsenelement 8, 9 lediglich um beispielhafte Bestandteile des erfindungsgemäßen Linsenimplantats handelt und dass diverse Abwandlungen des Basiselements 2, 3 und des Linsenelements 8, 9 vorgenommen werden können. Beispielsweise können mehr oder weniger als drei Befestigungsstifte 10 und Öffnungen 6 bzw. mehr oder weniger als drei Ösenelemente 7 auf der Vorderfläche des Basiselements 3 und Vorsprünge 11 an dem Linsenelement 9 bzw. mehr oder weniger als drei Ösenelemente 5 auf der Rückfläche und Vorsprünge 15 an dem weiteren Linsenelement 13 vorgesehen sein. Ferner kann auch das Basiselement 2 Befestigungsstifte aufweisen und das Linsenelement 8 zugehörige Öffnungen zum Einführen der Befestigungsstifte aufweisen. Es können auch sowohl das Basiselement 2 als auch das Linsenelement 8 entsprechende Befestigungsstifte und Öffnungen aufweisen. Ferner können anstatt des Befestigungsstifts 10 und der zugehörigen Öffnung 6 alternative Befestigungselemente vorgesehen sein, welche dazu geeignet sind, das Linsenelement 8 an dem Basiselement 2 zu befestigen wie dies in Bezug auf die Figuren 3 bis 7 beispielhaft illustriert ist. Ferner kann auch das Basiselement 3 Vorsprünge 11, 15 aufweisen und das Linsenelement 9 zugehörige Ösenelemente 5, 7 zum Aufnehmen der Vorsprünge 11, 15.

## Patentansprüche

1. Linsenimplantat zum Implantieren in ein zu behandelndes Auge, umfassend:
ein Basiselement (3); und
ein optisch wirksames Linsenelement (9),
wobei das Basiselement (3) plattenförmig ist und eine Vorderfläche und eine der Vorderfläche gegenüberliegende Rückfläche aufweist, wobei sich das Basiselement (3) in einer Ebene erstreckt oder eine Wölbung aufweist, welche einer Wölbung einer Hornhaut oder eines Linsensacks des zu behandelnden Auges folgt,
**dadurch gekennzeichnet, dass**
das Basiselement (3) die Form eines Ringsegments aufweist, wobei das Ringsegment eine Öffnung aufweist, die mehr als 5 % eines vollständig geschlossenen Rings beträgt,
wobei das Basiselement (3) mindestens ein an der Vorderfläche angeordnetes ösenförmiges Halteelement (7) zum Befestigen des Linsenelements (9) an dem Basiselement (3) aufweist, und
wobei das Linsenelement (9) mindestens einen Vorsprung (11) zum Einführen in das mindestens eine ösenförmige Halteelement (7) aufweist.

2. Linsenimplantat nach Anspruch 1, wobei der Vorsprung (11) dazu eingerichtet ist, nach dem Einführen des Vorsprungs (11) in das ösenförmige Halteelement (7) des Basiselements (3) durch eine Temperaturveränderung oder ein Befeuchten des Vorsprungs (11) aufzuquellen, um einen Kraftschluss zwischen dem Vorsprung (11) und dem ösenförmigen Halteelement (7) zu erhöhen.

3. Linsenimplantat nach Anspruch 1 oder 2, wobei die Rückfläche des Basiselements (3) zumindest ein weiteres ösenförmiges Halteelement (5) aufweist.

4. Linsenimplantat nach einem der Ansprüche 1 bis 3, wobei das Basiselement (3) eine Mehrzahl an ösenförmigen Halteelementen (7) aufweist.

5. Linsenimplantat nach einem der Ansprüche 1 bis 4, wobei das Basiselement (3) scharfkantige Außenkanten mit einem Kantenwinkel von maximal 90° aufweist.

6. Linsenimplantat nach einem der Ansprüche 1 bis 5, wobei das Basiselement (3) mindestens einen Vorsprung (4) aufweist, welcher sich radial in Richtung eines Mittelpunkts des Ringsegments erstreckt und wobei der Vorsprung (4) das ösenförmige Halteelement (7) zum Einführen des Vorsprungs (11) des Linsenelements (9) aufweist.

7. Linsenimplantat nach einem der Ansprüche 1 bis 6, wobei das Basiselement (3) Polymethylmethacrylat, PMMA, aufweist.

8. Linsenimplantat nach einem der Ansprüche 1 bis 7, wobei das Basiselement (3) eine lichtundurchlässige Beschichtung aufweist.

9. Linsenimplantat nach einem der Ansprüche 1 bis 8, wobei das Linsenelement (9) kreisförmig ist.

10. Linsenimplantat nach einem der Ansprüche 1 bis 9, wobei das Linsenelement (9) hydrophobes Acrylat aufweist.

## Claims

1. Lens implant for implantation in an eye to be treated, comprising:
a base element (3); and
an optically effective lens element (9),
wherein the base element (3) is plate-shaped and has a front surface and a rear surface opposite the front surface, wherein the base element (3) extends in a plane or has a curvature which follows a curvature of a cornea or a lens bag of the eye to be treated,
**characterized in that**
the base element (3) has the shape of a ring segment, the ring segment having an opening which is more than 5 % of a completely closed ring,
wherein the base element (3) has at least one eyelet-shaped retaining element (7) arranged on the front surface for fastening the lens element (9) to the base element (3), and
wherein the lens element (9) has at least one projection (11) for insertion into the at least one eyelet-shaped retaining element (7).

2. Lens implant according to claim 1, wherein the projection (11) is configured to swell after insertion of the projection (11) into the eyelet-shaped retaining element (7) of the base element (3) by a change in temperature or moistening of the projection (11) in order to increase a frictional connection between the projection (11) and the eyelet-shaped retaining element (7).

3. Lens implant according to claim 1 or 2, wherein the rear surface of the base element (3) has at least one further eyelet-shaped retaining element (5).

4. Lens implant according to any one of claims 1 to 3, wherein the base element (3) comprises a plurality of eyelet-shaped retaining elements (7).

5. Lens implant according to any one of claims 1 to 4, wherein the base element (3) has sharp-edged outer edges with an edge angle of at most 90°.

6. Lens implant according to any one of claims 1 to 5, wherein the base element (3) comprises at least one projection (4) which extends radially in the direction of a center point of the ring segment and wherein the projection (4) comprises the eyelet-shaped retaining element (7) for inserting the projection (11) of the lens element (9).

7. Lens implant according to any one of claims 1 to 6, wherein the base element (3) comprises polymethyl methacrylate, PMMA.

8. Lens implant according to any one of claims 1 to 7, wherein the base element (3) has an opaque coating.

9. Lens implant according to any one of claims 1 to 8, wherein the lens element (9) is circular.

10. Lens implant according to any one of claims 1 to 9, wherein the lens element (9) comprises hydrophobic acrylate.

## Revendications

1. Implant de lentille destiné à être implanté dans un oeil à traiter, comprenant :
un élément de base (3) ; et
un élément de lentille optiquement efficace (9),
dans lequel l'élément de base (3) est en forme de plaque et comprend une surface avant et une surface arrière opposée à la surface avant, l'élément de base (3) s'étendant dans un plan ou comprenant une courbure qui suit une courbure d'une cornée ou d'un sac cristallinien de l'oeil à traiter,
**caractérisé en ce que**
l'élément de base (3) présente la forme d'un segment d'anneau, lequel segment d'anneau comprenant une ouverture qui est supérieure à 5 % d'un anneau complètement fermé,
dans lequel l'élément de base (3) comprend au moins un élément de retenue en forme d'oeillet (7) disposé sur la surface avant pour fixer l'élément de lentille (9) à l'élément de base (3), et
dans lequel l'élément de lentille (9) comprend au moins une saillie (11) pour l'introduction dans ledit au moins un élément de retenue en forme d'oeillet (7).

2. Implant de lentille selon la revendication 1, dans lequel la saillie (11) est conçue pour gonfler après l'introduction de la saillie (11) dans l'élément de retenue en forme d'oeillet (7) de l'élément de base (3) par un changement de température ou une humidification de la saillie (11) afin d'augmenter une adhérence entre la saillie (11) et l'élément de retenue en forme d'oeillet (7).

3. Implant de lentille selon la revendication 1 ou 2, dans lequel la surface arrière de l'élément de base (3) comprend au moins un autre élément de retenue en forme d'oeillet (5).

4. Implant de lentille selon l'une quelconque des revendications 1 à 3, dans lequel l'élément de base (3) comprend une pluralité d'éléments de retenue en forme d'oeillet (7).

5. Implant de lentille selon l'une quelconque des revendications 1 à 4, dans lequel l'élément de base (3) comprend des arêtes extérieures à angles vifs avec un angle d'arête de 90° maximum.

6. Implant de lentille selon l'une quelconque des revendications 1 à 5, dans lequel l'élément de base (3) comprend au moins une saillie (4) qui s'étend radialement en direction d'un centre du segment d'anneau et dans lequel la saillie (4) comprend l'élément de retenue en forme d'oeillet (7) pour l'introduction de la saillie (11) de l'élément de lentille (9).

7. Implant de lentille selon l'une quelconque des revendications 1 à 6, dans lequel l'élément de base (3) comprend du polyméthacrylate de méthyle, PMMA.

8. Implant de lentille selon l'une quelconque des revendications 1 à 7, dans lequel l'élément de base (3) comprend un revêtement opaque.

9. Implant de lentille selon l'une quelconque des revendications 1 à 8, dans lequel l'élément de lentille (9) est circulaire.

10. Implant de lentille selon l'une quelconque des revendications 1 à 9, dans lequel l'élément de lentille (9) comprend de l'acrylate hydrophobe.
